# EUROPEAN PATENT SPECIFICATION

(11) **EP 0 707 498 B1**
(45) Date of publication and mention of the grant of the patent: **25.06.2003**
(21) Application number: 94919766.9
(22) Date of filing: 05.07.1994
(51) Int. Cl.: A61L 27/00, C12M 3/04

(54) **IMPLANTABLE PROSTHESIS, KIT AND DEVICE FOR MANUFACTURING THE SAME**
IMPLANTIERBARE PROTHESE, KIT UND VORRICHTUNG ZU DEREN HERSTELLUNG
PROTHESE IMPLANTABLE, TROUSSE ET DISPOSITIF DE FABRICATION

(30) Priority: 07.07.1993 GB 9314057; 07.07.1993 GB 9314471; 07.07.1993 GB 9314056; 14.07.1993 GB 9314580; 02.10.1993 GB 9320352; 02.10.1993 GB 9320368
(43) Date of publication of application: 24.04.1996
(73) Proprietor: Smith & Nephew PLC, London WC2N 6LA (GB)
(72) Inventor: WOLOWACZ, Sorrel Elizabeth, York YO2 5NH (GB); CARTER, Andrew James, Essex CB11 4 AL (GB); SEARLE, Richard John, York YO2 6HE (GB); MATTHEWS, Jane, Bridget, York YO2 1DP (GB); KING, John B., Bromley Kent BR1 2NW (GB); PALMER, Debra, Dorset SP7 8PW (GB); SHELTON, Julia, Caroline, Hackney London E9 7ND (GB)
(74) Representative: Gilholm, Stephen Philip
(86) International application number: GB9401455
(87) International publication number: WO95001810

(56) References cited:
- WO-A-85/04185
- WO-A-88/03785
- WO-A-90/12603
- WO-A-92/15259
- WO-A-93/07913
- WO-A-93/08850
- WO-A-93/19701

## Description

The present invention relates to methods suitable for replacing or repairing broken or damaged connective tissue such as ligaments or tendons and to prostheses for use in such methods. Also disclosed is a device for use in forming such prostheses, as well as kits from which the prostheses can be formed.

It is known from United States Patent No. US5078744 to repair damaged ligaments such as the anterior cruciate ligament (ACL) by replacing part of the damaged ligament by a prosthetic ligament comprising purified connective animal tendon or ligament tissue fibres which are cross-linked and formed into groups of aligned fibres.

The most common method of repair or reconstruction of the ACL is to implant a prosthetic graft comprising autogenous tissues. Thus it is common surgical practice to harvest autogenous tissue eg. patellar tendon from the host and to form a prosthesis for implantation.

A number of synthetic non-bioresorbable materials have been used in the manufacture of prosthetic ligaments, the materials being chosen for their affinity for supporting or encouraging the ingrowth of fibroblasts, after implantation of the prosthesis.

According to the present invention there is provided an implantable prosthesis which, in a form prior to implantation comprises a matrix of biocompatible, synthetic, bioresorbable polymeric material seeded with fibroblasts characterised in that the matrix has, in intimate contact therewith, a polymeric gel which fills the matrix and wherein the gel has the fibroblasts disposed therein.

By "synthetic", is merely meant a material which is not used naturally by the mammalian body in connective tissue repair or which is not a chemically modified form of such a material. Thus this term excludes collagen and artificially cross-linked collagen matrixes (although, if desired, collagen can be used in addition to the synthetic material).

The term "fibroblast" includes cells which are sometimes referred to as fibroblast, fibrocyte, tenocyte or synovioctye cells. This term also covers precursor cells to any of these cells.

By "substantially bioresorbable matrix material" is meant a three dimensional structure for supporting fibroblasts (which may be in the form of a scaffold, mesh or solid structure, for example) and which, in the implanted prosthesis, degrades substantially over time in a mammalian body, due to the chemical/biological action of body components (as opposed to simply breaking due to physical strain on to the prosthesis). Desirably after the prosthesis has been implanted in an adult human for five years (more preferably after only one year's implantation) the bioresorbable material will have degraded to such an extent so that it makes no substantial contribution to the structural integrity of the prosthesis.

Preferably the matrix may additionally comprise one or more of the following molecules: proteoglycans, glycosaminoglycans, fibronectin or its active binding domain, or one or more growth factors e.g. bone morphogenetic protein (BMP) fibroblast growth factor, angiogensis factor or other stimulatory factors.

In a further embodiment of the invention, the prosthesis or part thereof (e.g. area(s) of the prosthesis which will come into contact with bone after implantation), may be impregnated with osteoinductive or osteoconductive agents, to enable more easy infiltration by bone cells. Examples of suitable osteoinductive materials susceptible to infiltration include hydroxyapatite, freeze-dried or demineralised bone, growth factors (e.g. bone morphogenetic protein) etc. Impregnation may suitably be just before implantation of the prosthesis. Aptly such materials are incorporated into ends of the prosthesis.

In a further embodiment of the present invention, we make available a method of repairing or replacing damaged connective tissue in a human or non-human animal comprising the steps of:
incubating a biocompatible, synthetic, substantially bioresorbable matrix material in the presence of a suitable culture medium and of fibroblasts under suitable conditions for fibroblast seeding on or in the matrix and thereafter implanting the seeded matrix into a host.

Examples of suitable substantially bioresorbable synthetic polymers include polylactide (PLA), polyglycolide (PGA), polydioxanone, poly caprolactone (PCL), polyhydroxybutyrate (ICI BIOPOL™), polyhydroxybutyrate-co-hydroxyvalerate (ICI BIOPOL™), polyanhydrides, polyorthoesters, polyorthocarbonates, polyaminocarbonates, polytrimethylene carbonate and co-polymers incorporating monomers from which the aforesaid polymers can be formed.

When the prosthesis according to the present invention comprises a copolymer, the copolymer may incorporate hydroxyvalerate and hydroxybutyrate monomers. In such copolymers the amount of hydroxyvalerate present may be from 1 to 47% mol. Other particularly suitable copolymers are PLA/PGA and PLA/PCL copolymers.

Composites of a plurality the above substantially bioresorbable materials may also be suitable as or as part of the matrix material.

The matrix may be fabricated of two or more distinct materials (e.g. distinct fibre types) with different degradation rates, providing a two or more phase loss of mechanical properties with time. Also, the different fibre types may possess different mechanical properties. For example, highly extendable fibres may be combined with less extendable fibres. The matrix may be designed to elongate to a specified extent before the less extendable fibres prevent further extension. This design may be advantageous in exposing the cells to limited and controlled strain while protecting against damage to the forming tissue. For example, polycaprolactone fibres have a lower Young's modulus than polylactide fibres.

Furthermore, one polymer may be coated with another polymer. This is advantageous where the material of choice on the basis of mechanical properties is not necessarily the material of choice for cell culture (unless it is modified). Here a more biocompatible polymer may be used to coat a less biocompatible base material. For example, polylactide provides a better substrate for fibroblast proliferation than polycaprolactone. Polycaprolactone fibres could be coated with polylactide to improve compatibility with fibroblasts.

As indicated above, copolymeric materials may be used. This can be advantageous where the copolymers possess degradation rates intermediate between the rates of the homopolymers of which they are composed. Therefore, the degradation rate may be controlled by controlling the composition of the copolymer. Also, production of copolymer fibres by fibre spinning or extrusion may yield fibres with mechanical properties superior to those of homopolymers. Polylactide-Polyglycolide copolymers are good examples of both of these points.

Suitable fibroblasts for use in seeding the matrix may be autogenic fibroblasts, allogenic fibroblasts or xenogenic fibroblasts. Preferably, the fibroblasts are autogenic. The fibroblasts may originate from for example the dermis, tendons or ligaments. The fibroblasts for use in seeding the matrix may comprise a mixture of one or more of the above types of fibroblasts. Where the fibroblasts are autogenic, it is preferable to isolate them from the dermis, as this avoids the need for extensive invasive surgery.

The fibroblasts may be obtained according to any suitable method. A preferred method is by carrying out a skin biopsy.

The matrix material may be seeded with fibroblasts by placing the matrix in a culture vessel containing an appropriate culture medium (e.g. DMEM), in the presence of fibroblasts and incubating under cell culture conditions. The fibroblasts may be suspended in the culture medium and the resultant suspension added to the culture vessel either before or after addition of the matrix. The number of fibroblasts/ml of medium may be varied according to the degree of seeding it is desired to establish.

The prosthesis of the present invention may be used to either partially or totally replace a damaged ligament, tendon, cornea, dermis, dura (or other body part comprising connective tissue). Where the damage is substantial, the damaged ligament or tendon may be totally surgically replaced by the prosthesis. Where the damage is less substantial the matrix may be designed so as to be joined (e.g. by suturing) to the existing damaged ligament or tendon.

The matrix may be designed according to any one of a number of possibilities. Aptly the matrix is a fibrous structure. It may have loops or other structures at each end for aiding fixation to bone (using for example either the "two tunnel" or the "over-the-top" technique). It may be formed by any appropriate technique - e.g. braiding, knitting, weaving, crocheting etc. The matrix is desirably in elongate form and is preferably flexible.

The device may closely mimic the natural structure and fixation of the ligament or tendon. For example, for ACL reconstruction, the device could be composed of a hierarchy of fibres bundled together in fasicular units, passing directly from the femur to the tibia or taking a spiral path around the axis of the device. Fixation may be to the natural fixation areas of the ligament or tendon. Any appropriate fixation means may be used (e.g. screws, nails, staples or sutures). The fixation means may itself be bioresorbable, for example it may be formed of polyhydroxybutyrate.

On incubation under suitable conditions, the fibroblasts will grow on and/or in the matrix, thus producing a matrix seeded with fibroblasts.

The kit may additionally comprise a suitable medium for the proliferation of fibroblasts.

Ideally the kit is presented in a sterile package. Alternatively the parts of the kit may be sterilised just before use. Prior to implantation, the components of the kit can be incubated together under appropriate culture conditions as above described to allow the fibroblasts to seed the prosthesis.

The fibroblasts may be in any suitable form ready for use. Thus aptly the fibroblasts may be cryopreserved.

The matrix, or components/precursors thereof, may be provided in lyophilised form.

The present invention comprises, an implantable prosthesis which in a form prior to implantation comprises a biocompatible synthetic substantially bioresorbable matrix material having a polymeric gel in intimate contact therewith, the gel having fibroblasts dispersed therein. This is advantageous in that the gel can support the cells in a true three-dimensional arrangement rather than merely supporting a monolayer on the surface of a material. The environment closely mimics the natural physiological environment of the cells. Also, incorporation of cells in a gel can provide for even cell distribution, preventing cells from pooling which might otherwise occur due to gravitational influence.

The present invention makes available a method of repairing or replacing connective tissue in a human or other animal, comprising the steps of: incubating a biocompatible matrix material in the presence of a gel-forming composition and of fibroblasts under suitable conditions to form a prosthesis comprising a matrix contacting a polymeric gel, the gel having fibroblasts dispersed therein, and thereafter implanting the prosthesis into a host.

The present invention further provides a method of manufacturing the prosthesis as hereinbefore described comprising the step of incubating said matrix in the presence of a suitable culture medium, a gel-forming composition, fibroblasts and, if necessary, a gelation agent.

Suitable gel forming compositions include collagen gel forming compositions and fibrin gel forming compositions.

Fibroblasts in a collagen gel are capable of utilising the collagen and reorganising it. Under an appropriate mechanical stimulus they are capable of reorganising the fibrils into non-randomly orientated, organised structures resembling the natural ultrastructure of ligament and tendons. A mechanical stimulus may be the prevention of gel contraction which would otherwise occur over time by fixing the gel at two points. The matrix may be designed to achieve this. Alternatively, the gel on or in the matrix may be exposed to applied strain using a mechanised straining device to stimulate fibroblast alignment.

The method may comprise an additional step of incubating a gel-contacting matrix under suitable conditions for fibroblast proliferation in the gel and thereafter implanting the matrix into a host.

In the preferred embodiment of the present invention, the matrix is seeded by means of incubating the matrix in the presence of a suitable culture medium, a gel-forming composition and the fibroblasts to be seeded. An appropriate agent for causing gelation of the gel forming composition may also be used, if necessary.

Seeding the matrix in the presence of a gel-forming composition, fibroblasts (and a gelling agent, if required) results in a filled matrix, the gel having fibroblasts dispersed therein. The gel can be formed by the interaction of the gelling agent and the gel-forming composition. A preferred gel is a collagen gel. A Type I, II or III collagen solution may be prepared using an appropriate source of collagen. Thus for example a Type I collagen solution may be prepared from dermis (Type I collagen forms up to 70% of extracellular protein found in skin) as above described. Alternatively a Type I collagen solution may be prepared tendons, e.g. rat or bovine tendons, which comprise almost exclusively Type 1 collagen. The collagen may be extracted according to any of the standard methods known by those skilled in the art.

There are a number of suitable ways of incorporating the gel in or on the matrix. For example, the matrix may be suspended in such a manner that the gel-forming solution (optionally comprising fibroblasts) completely surrounds the matrix. A mould which surrounds the matrix may be used. Centrifugation or suction may alternatively be used to direct gel towards the matrix.

A prosthesis as herein described in kit form comprising a biocompatible matrix, a gel-forming composition and a source of fibroblasts.

Alternatively the kit may comprise a biocompatible matrix having a coating comprising a polymeric gel and/or having a polymeric gel incorporated therein and a source of fibroblasts. On incubation under suitable conditions the fibroblasts can invade the gel, thus producing a matrix bearing a gel having fibroblasts therein.

The prosthesis of the present invention offers an advantage over previously known prostheses which were designed to enhance ingrowth of fibroblasts after implantation and act as a scaffold through which fibroblasts can grow and form a new ligament, since it comprises fibroblasts prior to implantation. Thus the damaged tissue may be replaced by a prosthesis comprising viable fibroblasts which may be replicating. The fibroblasts may already substantially be aligned on implantation or at least oriented in a non-random manner. This process is speedier than previously known methods which rely on infiltration of prostheses by fibroblasts after implantation. It will be clear that the prosthesis may be implanted after an initial predetermined incubation period timed to result in seeding of the prosthesis with fibroblasts. Alternatively the prosthesis may be incubated for a longer incubation period than the initial incubation period so that the fibroblasts will be replicating and will have already started to secrete collagen fibrils when the prosthesis is implanted.

The prosthesis and method of the present invention offers other advantages over the common surgical practice or harvesting host patellar tendon in that it avoids the need for carrying out an extensive surgical operation to harvest the tendon. A simple skin biopsy (a standard procedure which does not result in substantial scarring) can be used to obtain fibroblasts which can then be proliferated in culture. In addition, the prosthesis can be designed to optimise fibroblast orientation. The cumulative effect of these advantages can result in a reduction in the length of a hospital stay.

In one preferred embodiment of the present invention, where a collagen gel contacts the matrix, the prosthesis of the present invention provides a source of collagen which can be used by the fibroblasts. The collagen in the gel is preferably in a non-cross-linked form.

Materials suitable for use in a matrix of the present invention can be assessed as exemplified below:-

### Assessment of Materials

In order to make an initial assessment of suitable materials for supporting fibroblast growth, various materials were obtained (which are not to be construed as limiting), as indicated in Table 1 below, and moulded into films for 5min at 2.5 Tons at the following temperatures: Polylactide 170°C; polyglycolide, 245°C; polyhydroxybutyrate, 185°C; and polycaprolactone, 65°C.

**Table 1**

| Material | Supplier | Fig. |
|---|---|---|
| Polylactide | Medisorb, Cincinnati, Ohio, USA | 1a |
| Polyglycolide | Medisorb, Cincinnati, Ohio, USA | 1b |
| Polyhydroxybutyrate | Goodfellows, Cambridge, UK 1c | |
| Polycaprolactone | Birmingham Polymers Inc. Alabama, USA | 1d |

Fibroblasts were seeded onto the surfaces of these materials at a density of 1 x 10⁴ cells/cm² of material and incubated for 3 days under culture conditions.

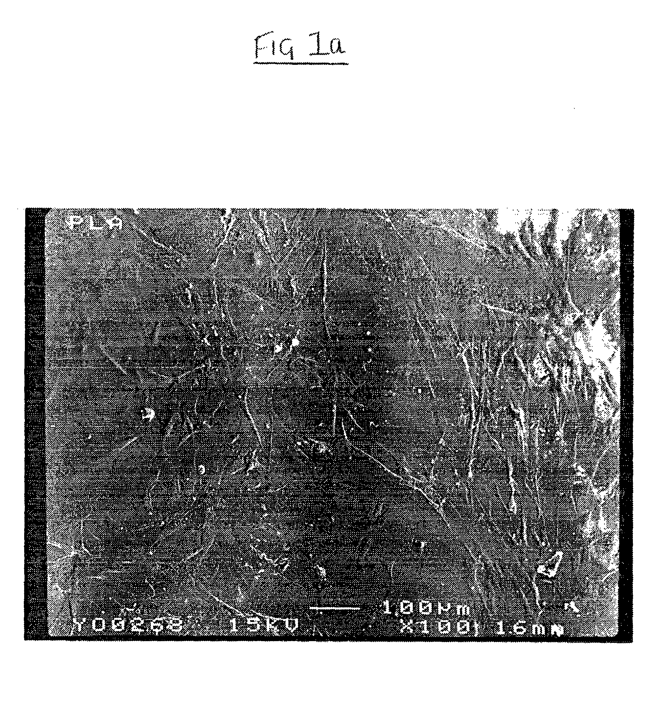

After the incubation period, photomicrographs were taken of the cell-seeded samples. These are shown in Figs. 1a to 1d for the samples indicated in Table 1 above (photocopies of all of the photographs provided for this application are provided immediately after the relevant photographs).

Fibroblasts can be seen to be well adhered to the surfaces of all of the materials and to exhibit the morphology typical of healthy cultural fibroblasts.

One sample of fibroblasts was grown on polylactide as described above apart from the fact that a longer (16 day) culture period was used.

After this period the cells were stained with a viable stain (calcein AM (2µM)) and visualised by fluorescence microscopy using a fluorescein filter. A confluent monolayer of viable cells was observed, showing that polylactide is capable of supporting viable fibroblasts for extended periods of culture.

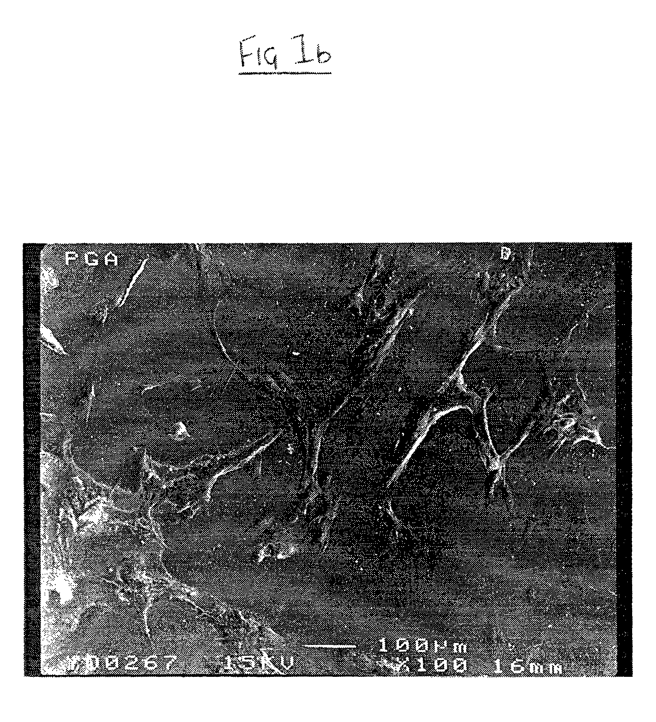

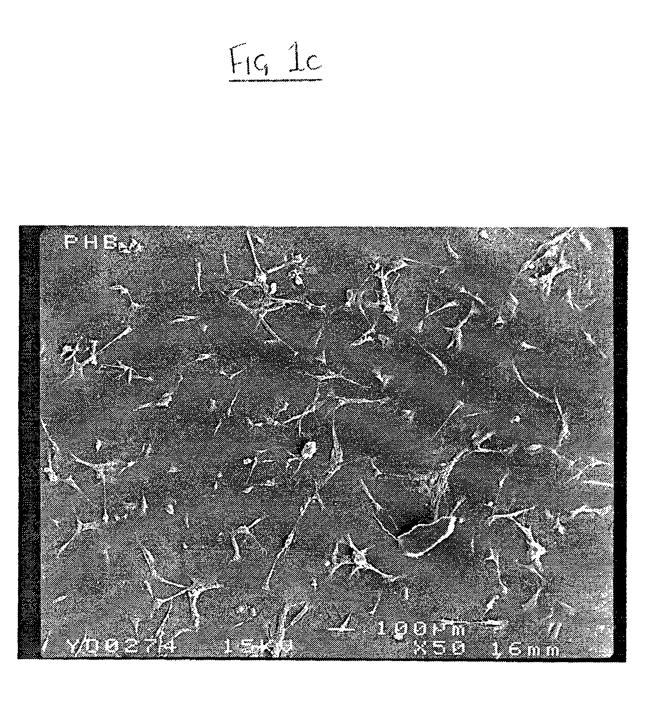

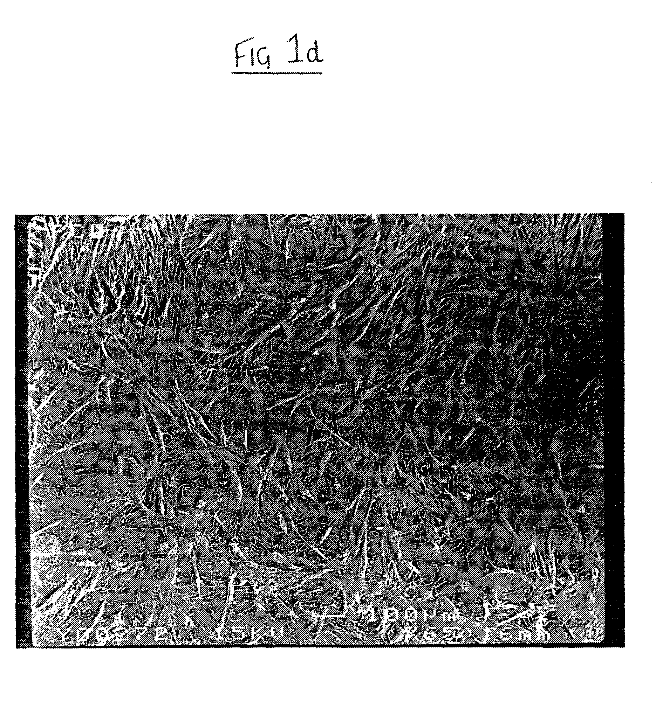

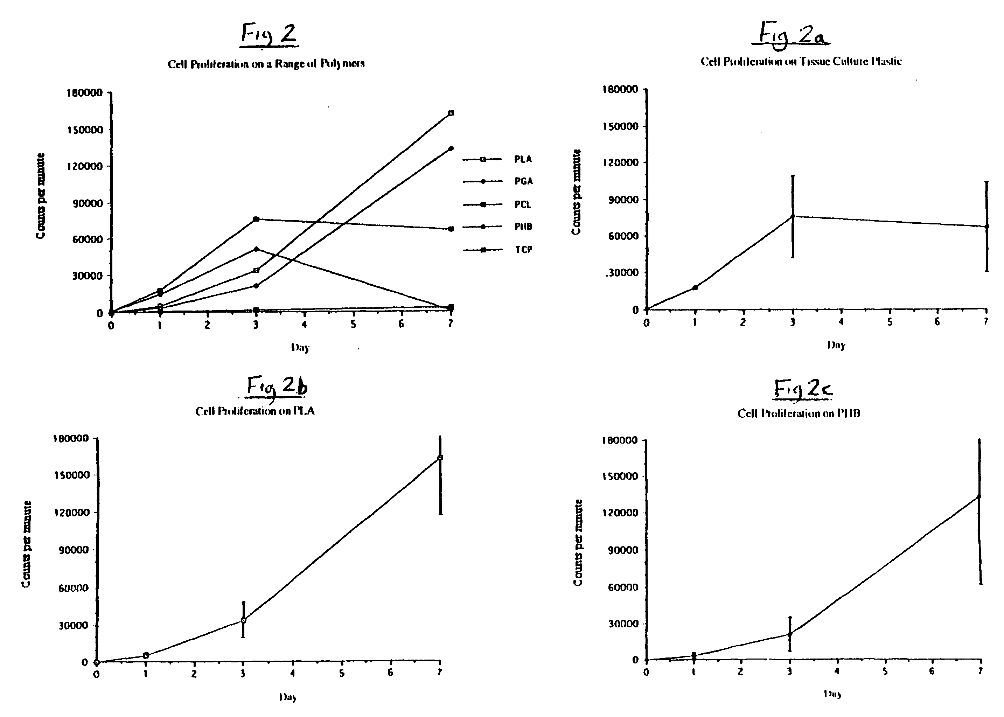

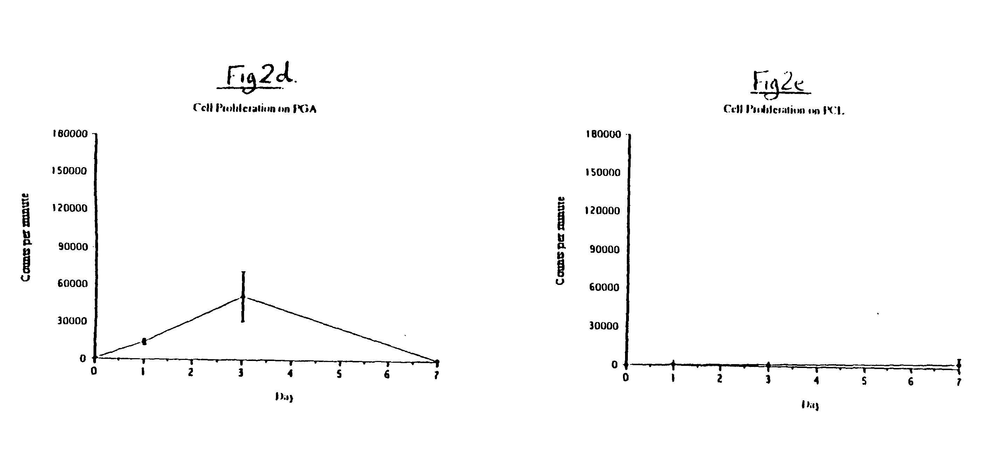

Figure 2 is a graph showing the relative rate of proliferation of fibroblasts on four examples of bioresorbable synthetic materials:polylactide (PLA); polyglycolide (PGA) polyhydroxybutyrate (PHB) and polycaprolactone (PCL) (all as described above) in comparison with a tissue culture treated polystyrene (TCP) control (since TCP is known to support good fibroblast growth).

Figures 2a) to e) show each of these materials on a single graph (for each of reference). Cells were seeded at 1 x10⁴ cells.cm⁻² in triplicate and the rate of proliferation determined by measuring the uptake of tritiated thymidine into cellular DNA at timepoints up to 7 days after incubation using standard cell culture techniques. The medium was changed at 2, 4 and 6 days. The points represent the mean of three determinations and the error bars represent the range. All polymers supported fibroblast proliferation.

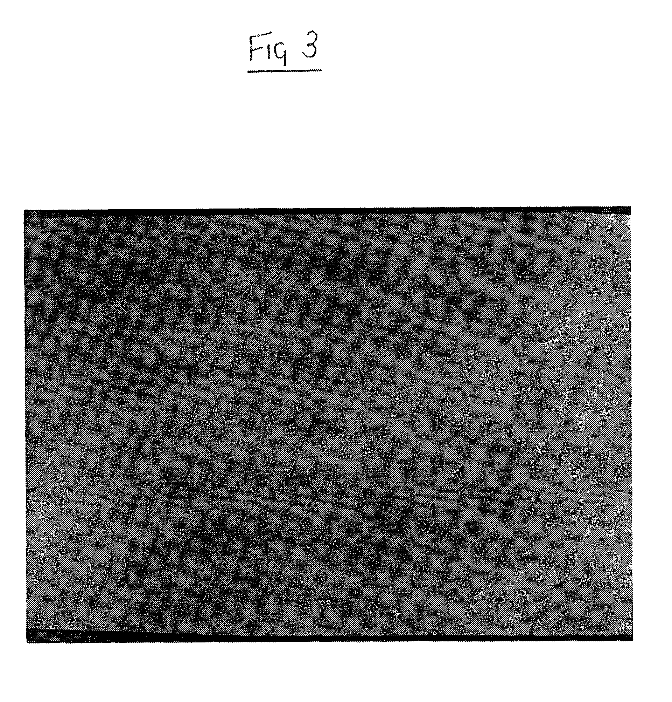

Figure 3 is a photomicrograph of fibroblasts embedded within a three dimensional collagen gel after 15 days of culture. The cell-seeded gel was prepared as described in example 2 (which will be described later) apart from the fact that it was not used to contact a matrix. The gel provides a three-dimensional structure in which the cells are embedded and can form interactions with collagen molecules via membrane integrin receptors. The cells are randomly arranged, exhibit long processes and are capable of reorganising collagen fibrils within the gel.

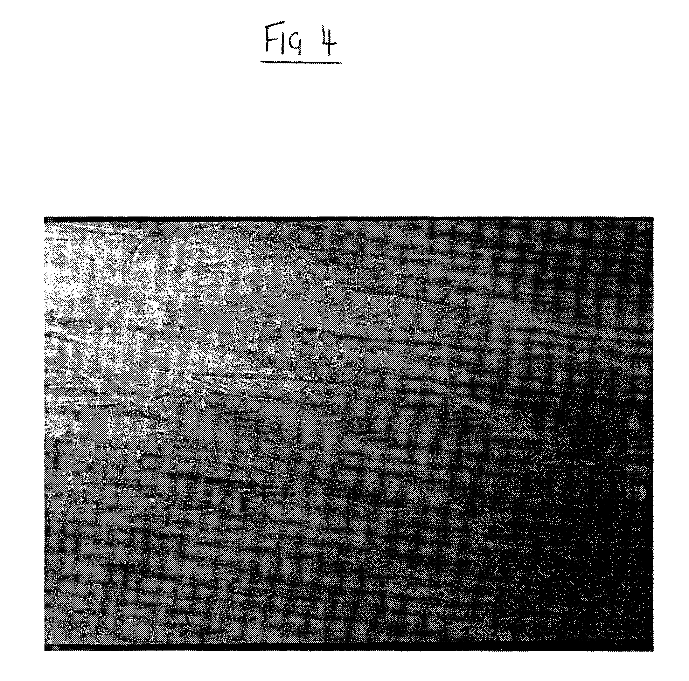

Figure 4 is a photomicrograph of fibroblasts embedded within a three dimensional collagen gel as described for Fig. 3 above, apart from the fact that the gel has now been constrained from contracting in one direction by two stainless steel pegs glued to a culture dish with a tissue culture compatible adhesive. The cells are arranged in a highly orientated fashion, their long axes being parallel to the axis between the contraining pegs. The collagen fibrils align along the same axis. This effect is due to the pegs preventing the gel contracting, as would otherwise occur in the presence of fibroblasts in culture.

The following examples, which are not to be construed as limiting, illustrate how various cell-seeded matrixes can be produced.

### a) Preparation of Cells

A biopsy is washed three times in phosphate buffered saline (PBS), and rinsed in 70% alcohol. The rinsed biopsy is then dipped into Dulbecco's Modified Essential Medium (DMEM) and incubated at 37°C for 24 hours. After incubation, the biopsy is cut into small pieces under PBS. The cut pieces are transferred to a 50mm petri dish, containing about 5ml of collagenase solution to allow digestion. The epidermal sheets are removed from the collagenase solution. The resultant solution is centrifuged. The fibroblast cell pellet is resuspended in DMEM and thereafter seeded in a 35mm petri dish using DMEM. The cells may be confluent in from 2-4 days. Thereafter the cells may be cultured to provide an appropriate quantity of fibroblasts for seeding the matrix.

A suitable medium for culturing the isolated fibroblasts may comprise DMEM which may be supplemented with the following:
glutamine, foetal calf serum, non essential amino acids and
antibiotics. In addition the medium may have a buffering agent such as bicarbonate.

### b) Preparation of matrix material

A polylactide matrix material suitable for use in a prosthesis for replacing a ligament can be prepared by obtaining polylactide fibres and then braiding them to form a braid of appropriate dimensions to replace the ligament.

Polylactide fibres can be obtained by extrusion, fibre spinning, melt-spinning, drawing, heat annealing etc.

Braiding of the fibres can be done by standard braiding techniques, the length and thickness of the braid, number of fibres present and diameter of fibres present being selected to form a braid with appropriate properties.

The ends of the device are constructed in a suitable way to aid fixation of the device by a screw or other fixation means. This is done by forming eyelets at the ends.

### c) Straining of the cell-seeded matrix

The cell seeded device is gripped at both ends in a straining apparatus which causes the device to be strained along a single longitudinal axis. This is done for sufficient time so as to cause the fibroblasts substantially to align along the general direction of the longitudinal axis, as can be assessed by microscopic analysis of the cells. The apparatus comprises a culture chamber so that straining can occur over several hours or even several days and yet the cells can remain viable. Typically the device is strained at 37°C.

During straining a culture medium is used to culture the cells under suitable conditions. This includes serum, ascorbate or stable analogues thereof, together with growth factors.

The ascorbate stimulates the fibroblasts to synthesise collagen; the serum contains factors promoting cell proliferation and cell adherence and the growth factors can stimulate cell proliferation, development and migration.

### d) Implantation of the cell-seeded matrix

Once the cell-seeded device has been strained for a sufficient period to obtain a desired degree of alignment of fibroblasts, it is removed from the cell-straining device and implanted into a patient by any desired technique.

Minimal invasive surgery is preferred. For a prosthetic anterior cruciate ligament implantation be done utilising the "two-tunnel" or the "over the top" techniques. Fixation can be achieved by using screws (or other fixation elements) placed through the eyelets of the matrix. The screws are suitably formed of a biodegradable material, such as polyhydroxybutyrate.

### Example 1 : Preparation of a fibroblast seeded polylactide matrix prosthesis comprising a collagen gel in which the fibroblasts are incorporated

This can be done in an analogous manner to the method described above, except for the inclusion of an alternative procedure whereby fibroblasts are incorporated in a collagen gel which is used to seed the polylactide matrix. This extra procedure is described below:-

### a) Preparation of collagen

It is desired to form the collagen in a form which is acid soluble and which is not cross-linked. This can be done as follows:-
Type I collagen is prepared from tendon but could be from other tissue e.g. skin. The tissue is minced finely, disinfected in 70% (v/v) ethanol for at least 30 mins, dispersed in acetic acid (1% v/v) and incubated with agitation at 4°C. The supernatant is removed and neutralised by addition of an appropriate volume of 1.0M sodium hydroxide. The precipitated type I collagen is pelleted by centrifugation at 8,000 x g and the pellet resuspended in an appropriate volume of acetic acid (1% v/v). The collagen concentration of this solution is determined by any appropriate method (e.g. a total protein assay - the BCA assay) and the concentration of the collagen solution adjusted appropriately (e.g. 3mg.ml⁻¹ can be used).
If the product is a kit, sterile collagen solution may be lyophilised and stored under vacuum or an inert gas (e.g. argon) to prevent cross-linking. A diluent (acetic acid) could also be provided. Alternatively, it could be provided as a solution and stored at 4°C to -20°C.

### b) Seeding of matrix material with cells

Three components are typically used to seed the matrix:- cells suspended in an appropriate medium, collagen solution and a gelling agent - (e.g. 1M sodium hydroxide). The final collagen concentration may be approximately 1 mg.ml⁻¹ and the seeding density approx. 3x10⁴ cells per ml of gel (or volume within the matrix). The three components are maintained at 0°C to 4°C, mixed and added to the matrix. Once the matrix is fully impregnated, it is incubated at 37°C and gelling is initiated.

The impregnation of the matrix may be achieved by any suitable method. The matrix may be merely immersed in the solution within a mould. Alternatively the solution may be sucked into the scaffold by use of a vaccum or forced in by centrifugation (for example within a mould centrifuged at 500rpm for 5min at 0-4°C).

The rate of setting of the gel may be varied by varying the temperature.

### Example 2: Preparation of a fibroblast seeded polylactide matrix prosthesis comprising a fibrin gel in which the fibroblasts are incorporated

This can be done by an analogous method to that described in Example 1, except that a fibrin gel rather than a collagen gel is used.

Again, three components are used: cell suspension, fibrinogen solution and thrombin solution containing calcium chloride (mM). The final cell concentration may be 3x10⁴ cells per ml, the fibrinogen concentration may be 3mg/ml, the thrombin activity 2.5 Units/ml and the calcium chloride concentration 5mM. The reagents are mixed and then incubated with the matrix. Once impregnated with the solution, the matrix is incubated at 37°C to allow rapid gelling. Impregnation of the matrix may be conducted by any of the techniques described above.

The rate of gelling may be varied by varying the thrombin activity and/or the temperature.

## Claims

1. An implantable prosthesis which in a form prior to implantation comprises a matrix of biocompatible, synthetic, substantially bioresorbable polymeric matrix material seeded with fibroblasts **characterised in that** said matrix has, in intimate contact therewith, a polymeric gel which fills said matrix and wherein said gel has said fibroblasts dispersed therein.

2. A prosthesis according to claim 1 wherein the gel is a collagen gel.

3. A prosthesis according to claim 1 wherein said gel is a fibrin gel.

4. A prosthesis according to claim 1 or 2 wherein said gel comprises Type I collagen.

5. A prosthesis according to any preceding claim wherein said fibroblasts are non-randomly oriented.

6. A prosthesis according to claim 5 wherein said fibroblasts are substantially aligned.

7. A prosthesis according to any preceding claim wherein the biorsesorbable matrix material comprises one or more of the following materials; a polylactide, a polyglycolide, a polydioxanone, a polycaprolactone, a polyhydroxybutyrate, a polyhydroxybutyrate-co-hydroxyvalerate, a polyanhydride, a polyorthoester, a polyothrocarbonate, a polyaminocarbonate, a polytrimethylene carbonate or a co-polymer which incorporates monomers from which the above mentioned polymers are formed.

8. A prosthesis according to claim 7 further comprising a non-bioresorbable matrix material.

9. A prosthesis according to claim 8 wherein said non-bioresorbable matrix material is a polyester, a polyethylene, a polypropylene, PTFE, carbon fibre or a composite of two of more of the aforesaid materials.

10. A prosthesis according to any preceding claim further comprising one or more of the following:- proteoglycans, glycosaminoglycans, fibronectin or its active binding domain, growth factors, osteoinductive and osteoconductive materials.

11. A prosthesis according to any preceding claim wherein said matrix material is flexible.

12. A prosthesis according to any preceding claim comprising a plurality of bioresorbable matrix materials having different rates of bioresorption.

13. A prosthesis according to any preceding claim wherein said prosthesis is in the form of a fibrous member.

14. A prosthesis according to any preceding claim which is in the form of a woven, knitted, crocheted or braided member.

15. A prosthesis according to any preceding claim in kit form comprising said matrix material, a source of fibroblasts and a composition for forming said gel.

16. A kit according to claim 15 further comprising a medium suitable for the proliferation of fibroblasts.

17. A method of manufacturing the prosthesis of claim 1 comprising the step of incubating said matrix in the presence of a suitable culture medium, a gel - forming composition, fibroblasts and, if necessary, a gelation agent.

18. A method of manufacturing the prosthesis according to claim 17 further comprising the step of fixing said gel at two points.

## Patentansprüche

1. Eine implantierbare Prothese, die in einer vor der Implantation gegebenen Form aus einer Matrix aus biokompatibler, synthetischer, im Wesentlichen bioresorbierbarer Polymermatrixsubstanz, die mit Fibroblasten angeimpft ist, besteht, **dadurch gekennzeichnet, dass** die Matrix ein Polymergel aufweist, mit dem sie in sehr gutem Kontakt steht und das die Matrix ausfüllt, und wobei das Gel die darin dispergierten Fibroblasten aufweist.

2. Prothese gemäß Anspruch 1, wobei das Gel ein Kollagengel ist.

3. Prothese gemäß Anspruch 1, wobei das Gel ein Fibringel ist.

4. Prothese gemäß Anspruch 1 oder 2, wobei das Gel aus Typ-I- Kollagen besteht.

5. Prothese gemäß einem der vorhergehenden Ansprüche, wobei die Fibroblasten nicht zufällig orientiert sind.

6. Prothese gemäß Anspruch 5, wobei die Fibroblasten im Wesentlichen ausgerichtet sind.

7. Prothese gemäß einem der vorhergehenden Ansprüche, wobei die bioresorbierbare Matrixsubstanz aus einer oder mehreren der folgenden Substanzen besteht; einem Polylactid, einem Polyglycolid, einem Polydioxanon, einem Polycaprolacton, einem Polyhydroxybutyrat, einem Polyhydroxybutyrat-co-hydroxyvalerat, einem Polyanhydrid, einem Polyorthoester, einem Polyorthocarbonat, einem Polyaminocarbonat, einem Polytrimethylencarbonat oder einem Copolymer, das Monomere, aus denen die oben genannten Polymere gebildet sind, einlagert.

8. Prothese gemäß Anspruch 7, ferner bestehend aus einer nicht bioresorbierbaren Matrixsubstanz.

9. Prothese gemäß Anspruch 8, wobei die nicht bioresorbierbare Matrixsubstanz ein Polyester, ein Polyethylen, ein Polypropylen, PTFE, Carbonfaser oder ein Verbundstoff aus zwei oder mehreren der bereits genannten Substanzen ist.

10. Prothese gemäß einem der vorhergehenden Ansprüche, ferner bestehend aus einem oder mehreren des Folgenden: Proteoglycanen, Glycosaminoglycanen, Fibronectin oder seinem Aktivbindungsbereich, Wachstumsfaktoren, osteoinduktiven und osteokonduktiven Substanzen.

11. Prothese gemäß einem der vorhergehenden Ansprüche, wobei die Matrixsubstanz flexibel ist.

12. Prothese gemäß einem der vorhergehenden Ansprüche, bestehend aus einer Vielzahl von bioresorbierbaren Matrixsubstanzen, die unterschiedliche Bioresorptionsraten aufweisen.

13. Prothese gemäß einem der vorhergehenden Ansprüche, wobei die Prothese die Form eines faserförmigen Elements aufweist.

14. Prothese gemäß einem der vorhergehenden Ansprüche, die die Form eines gewebten, gewirkten, gehäkelten oder geflochtenen Elements aufweist.

15. Prothese gemäß einem der vorhergehenden Ansprüche in Form eines Kits, bestehend aus der Matrixsubstanz, einer Quelle von Fibroblasten und einer Zusammensetzung zur Bildung des Gels.

16. Ein Kit gemäß Anspruch 15, ferner bestehend aus einem Medium, das zur Proliferation von Fibroblasten geeignet ist.

17. Ein Verfahren zur Herstellung der Prothese gemäß Anspruch 1, bestehend aus dem Schritt des Inkubierens der Matrix unter Anwesenheit eines geeigneten Kulturmediums, einer Gel bildenden Zusammensetzung, von Fibroblasten und, falls notwendig, eines Geliermittels.

18. Verfahren zur Herstellung der Prothese gemäß Anspruch 17, ferner bestehend aus dem Schritt des Befestigens des Gels an zwei Punkten.

## Revendications

1. Une prothèse implantable qui, dans une forme antérieure à l'implantation, comprend une matrice de matériau de matrice polymérique substantiellement biorésorbable, synthétique et biocompatible ensemencée de fibroblastes, **caractérisée en ce que** ladite matrice possède un gel polymérique qui remplit ladite matrice et avec lequel elle est en contact intime, et dans laquelle lesdits fibroblastes sont dispersés dans ledit gel.

2. Une prothèse selon la revendication 1 dans laquelle le gel est un gel de collagène.

3. Une prothèse salon la revendication 1 dans laquelle ledit gel est un gel de fibrine.

4. Une prothèse selon la revendication 1 ou la revendication 2 dans laquelle ledit gel comprend du collagène de Type I.

5. Une prothèse selon n'importe quelle revendication précédente dans laquelle lesdits fibroblastes ne sont pas orientés au hasard.

6. Une prothèse selon la revendication 5 dans laquelle lesdits fibroblastes sont substantiellement alignés.

7. Une prothèse selon n'importe quelle revendication précédente dans laquelle le matériau de matrice biorésorbable comprend au moins un des matériaux suivants ; un polylactide, un polyglycolide, un polydioxanone, un polycaprolactone, un polyhydroxybutyrate, un polyhydroxybutyrate - co-hydroxyvalérate, un polyanhydride, un polyorthoester, un polyorthocarbonate, un polyaminocarbonate, un carbonate de polytriméthylène ou un copolymère qui incorpore des monomères à partir desquels les polymères mentionnés plus haut sont formés.

8. Une prothèse selon la revendication 7 comprenant de plus un matériau de matrice qui n'est pas biorésorbable.

9. Une prothèse selon la revendication 8 dans laquelle le matériau de matrice qui n'est pas biorésorbable est un polyester, un polyéthylène, un polypropylène, du PTFE, de la fibre de carbone ou un composite d'au moins deux des matériaux susmentionnés.

10. Une prothèse selon n'importe quelle revendication précédente comprenant de plus au moins un des suivants : - des protéoglycanes, des glycosaminoglycanes, de la fibronectine ou son domaine de liaison actif, des facteurs de croissance, des matériaux ostéoinducteurs et ostéoconducteurs.

11. Une prothèse selon n'importe quelle revendication précédente dans laquelle ledit matériau de matrice est flexible.

12. Une prothèse selon n'importe quelle revendication précédente comprenant une pluralité de matériaux de matrice biorésorbables ayant différents taux de biorésorption.

13. Une prothèse selon n'importe quelle revendication précédente dans laquelle ladite prothèse est sous la forme d'un élément fibreux.

14. Une prothèse selon n'importe quelle revendication précédente, laquelle est sous la forme d'un élément tissé, tricoté, crocheté ou tressé.

15. Une prothèse selon n'importe quelle revendication précédente sous forme de kit comprenant ledit matériau de matrice, une source de fibroblastes et une composition pour former ledit gel.

16. Un kit selon la revendication 15 comprenant de plus un milieu approprié à la prolifération de fibroblastes.

17. Un procédé de fabrication de la prothèse de la revendication 1 comprenant l'étape consistant à incuber ladite matrice en présence d'un milieu de culture approprié, d'une composition de formation de gel, de fibroblastes et, si besoin, d'un agent de gélification.

18. Un procédé de fabrication de la prothèse selon la revendication 17 comprenant de plus l'étape consistant à fixer ledit gel au niveau de deux points.
